# EUROPEAN PATENT APPLICATION

(11) **EP 1 016 413 A1**
(43) Date of publication of application: **05.07.2000**
(21) Application number: 98124834.7
(22) Date of filing: 30.12.1998
(51) Int. Cl.: A61K 38/27, C12N 5/06, C12N 5/08, A61K 35/14, A61K 35/28

(54) **Human growth hormone to stimulate mobilization of pluripotent hematopoietic stem cells**

(71) Applicant: Applied Research Systems ARS Holding N.V., Curacao (AN)
(72) Inventor: Gianni, Allesandro Massimo, I-20135 Milano (IT)
(74) Representative: Dufresne, Guillaume Alain François

(57) **Abstract**

The invention relates to the field of hematopoietic CD34⁺ cell mobilization. In particular, this invention relates to a method for increasing the mobilization of pluripotent hematopoietic CD34⁺ cells by administration of human growth hormone or one of its derivates to the individual.

## Description

Bone marrow transplantation (BMT) is a clinical procedure in which pluripotent hematopoietic cells obtained from bone marrow are transplanted to a patient. BMT is the treatment of choice in several hematological disorders, including malignancies, Severe Combined Immune Deficiencies (SCIDs), congenitally or genetically determined hematopoietic abnormalities, anemia, aplastic anemia, leukemia and osteopetrosis (Fischer et al., 1998). In the last ten years, the use of BMT grew from less than 5'000 to more than 40'000 annually (Waters et al., 1998).

Under steady state condition, the majority of hematopoietic stem and progenitor cells reside in the bone marrow and only a low number of these cells are detectable in peripheral blood. However, additional stem cells can be mobilized into the peripheral blood by treatment with myelosuppressive agents and/or certain hematopoietic growth factors (Van Hoef, 1998). Studies have demonstrated that peripheral blood stem cells (PBSC) infused in a host exhibits enhanced potential for engraftment as compared to bone marrow-derived stem and progenitor cells (Gianni et al., 1989 ; Larsson et al. , 1998). Thus, PBSC mobilized by chemotherapy, hematopoietic growth factors or the combination of these modalities are currently used in both autologous and nonautologous transplantation settings (Van Hoef, 1998 ; Anderlini and Korbling, 1997). In the case of nonautologous transplantation, the donors of stem cells are normal individuals and the procedure for mobilization of stem cells into the blood stream has to be achieve with minimal discomfort. In this case, stem cells mobilization with hematopoietic growth factors is preferred to the treatment with antiblastic drugs (i.e. cyclophosphamid).

Several hematopoietic growth factors, such as G-CSF, EPO and CSF have been studied as mobilizing agents and are currently used to increase the number of PBSC prior to leukapheresis (Henry, 1997; Weaver and Testa, 1998). Treatments aimed at stimulating the overall hematopoiesis may be of great interest to mobilize a large set of progenitor cells and stem cells. Increased mobilization of stem cells is extremely valuable in the context of hematopoietic stem cells transplantation by reducing the number of leukapheresis required to collect sufficient amount of hematopoietic stem cells to be transplanted.

The first part of the invention provides a new mobilising agent used to increase the number of circulating cells capable of regenerating hematopoiesis in vivo in an individual.

The new mobilizing agent of the invention is growth hormone and especially Human Growth Hormone (hGH) or one of its derivatives or any factor inducing growth hormone release.

It has now been found that, by administering growth hormone and especially Human Growth Hormone (hGH) or one of its derivatives or any factor inducing growth hormone release, a mobilization of cells capable of regenerating hematopoiesis in vivo is obtained in the peripheral blood. Therefore, growth hormone and especially human Growth Hormone (hGH) or one of its derivatives or any factor inducing growth hormone release, administered alone or in combination with other factors, represents a new method or use to mobilize cells capable of regenerating hematopoiesis in vivo to the peripheral blood.

Human Growth Hormone (hGH), also known as somatotropin is a protein hormone produced and secreted by the somatotropic cells of the anterior pituitary. hGH plays a key role in somatic growth through its effects on the metabolism of proteins, carbohydrates and lipids. In addition to its effects on somatic growth, hGH has been shown to stimulate blood cells in vitro (Derfalvi et al., 1998 ; Merchav et al; 1988), to increase erythrocytes and hemoglobin counts (Valerio et al. , 1997 ; Vihervuori et al. , 1996), to enhance both proliferation and Ig production in plasma cell lines (Kimata and Yoshida, 1994) and to stimulate CD8⁺ cell counts and, to a lesser extent CD4⁺ cell counts (Geffner, 1997).

The methods and uses of the invention which use the mobilising agent of the invention have several advantages :
- There is a low number of circulating cells capable of regenerating hematopoiesis. This number is considered insufficient to provide a cells engraftment dose by single or multiple apheresis in a reasonable time period. Methods and uses of the invention solve this problem by a temporary peripheralization of said cells and subsets into the circulating blood which is widely used to significantly increase in the blood the yield of circulating cells capable of regenerating hematopoiesis in vivo, thus minimizing the number of aphereses needed to achieve an engraftment dose.
- Other advantages of the methods and uses of the invention include the possibility of :
   a) circumventing the need of general anasthesia,
   b) harvesting even if iliac bones are damaged by previous radiotherapy or infiltrated with malignant cells,
   c) achieving restoration of sustained hematopoietic functions more rapidly than with BM derived progenitor cells.
   d) achieving restoration of sustained hematopoietic functions more rapidly and effectively than without a pre-treatment including a method or a use of the invention.
- Generally, methods and uses of the invention are effective and safe to mobilize to peripheral blood cells capable of regenerating hematopoiesis in vivo.
- Methods and uses of the invention are not toxic in view of main parameters of toxicity which are for example tumor growth, clinical and instrumental symptoms, or laboratory tests for cardiac, liver and renal function.
- The increased mobilization of circulating cells capable of regenerating hematopoiesis in vivo obtained with the methods and uses of the invention is extremely valuable in the context of hematopoietic stem cells transplantation by reducing the number of leukapheresis required to collect sufficient amount of hematopoietic cells to be transplanted.
- Methods and uses of the invention lead to a reduction of the volume of blood required to be processed during the apheresis or leukapheresis procedure in order to obtain the specified target number of cells. The advantages of processing a reduced volume of blood are that the patient spends less time on the cell separating machine, that it reduces the toxicity of the procedure, particularly in terms of the volume of anticoagulant to which the patient would be exposed during the procedure, that it reduces the machine and the operator's time.
- Furthermore, the transplantation of a population of blood cells enriched with cells capable of regenerating hematopoiesis in vivo, which population is obtained from the peripheral blood by the methods or uses of the invention has the effect to enhance reconstitution of recipient's hematopoietic and immune systems following myeloablative or antiblastic therapies.

In a first aspect, the invention concerns a method of preparation of a population of circulating cells capable of regenerating hematopoiesis in vivo comprising :
a) administering to a donor a composition comprising growth hormone or one of its derivatives or any factor inducing growth hormone release in an amount sufficient to increase in said donor the number of circulating cells capable of regenerating hematopoiesis in vivo,
b) isolating a population of circulating cells capable of regenerating hematopoiesis in vivo from the peripheral blood of said donor.

The method of the invention thus produces a population of cells capable of regenerating hematopoiesis in vivo, this population being destined for transplantation in the same or in different individuals.

Thus, the invention concerns a method of preparation of a population of blood cells enriched with cells capable of regenerating hematopoiesis in vivo comprising :
a) administering to a donor a composition comprising growth hormone or one of its derivatives or any factor inducing growth hormone release in an amount sufficient to increase in said donor the number of circulating cells capable of regenerating hematopoiesis in vivo,
b) isolating a population of blood cells enriched with cells capable of regenerating hematopoiesis in vivo from the peripheral blood of said donor.

In a further embodiment, the invention relates to a method of isolating an increased number of circulating cells capable of regenerating hematopoiesis in vivo from a donor comprising :
a) administering to a donor a composition comprising growth hormone or one of its derivatives or any factor inducing growth hormone release alone, or in combination with other hematopoietic growth factors, to the subject in an amount sufficient to induce mobilization of cells capable of regenerating hematopoiesis in vivo to the peripheral blood,
b) isolating a population of blood cells enriched with cells capable of regenerating hematopoiesis in vivo from the peripheral blood of said donor.

In another embodiment, the invention concerns a method of preparation of a population of circulating cells capable of regenerating hematopoiesis in vivo comprising :
a) administering to a donor a composition comprising growth hormone or one of its derivatives or any factor inducing the growth hormone release in an amount sufficient to induce in said donor the mobilization or peripheralisation of circulating cells capable of regenerating hematopoiesis in vivo,
b) isolating a population of circulating cells capable of regenerating hematopoiesis in vivo from the peripheral blood of said donor or isolating a population of blood cells enriched with circulating cells capable of regenerating hematopoiesis in vivo from the peripheral blood of said donor.

Step b) [i.e. 〈〈 isolating a population of (blood cells enriched with) circulating cells capable of regenerating hematopoiesis in vivo from the peripheral blood of said donor 〉〉] of the methods or uses of the invention may correspond to the operation of removing peripheral blood from the donor wherein the number of cells capable of regenerating hematopoiesis in vivo has been increased by administration of growth hormone or one of its derivatives or any factor inducing growth hormone alone or in combination with other factors.

The operation of removing peripheral blood from the donor may correspond to leukapheresis. Leukapheresis is a procedure, in which, leukocytes are removed from the withdrawn blood and the remainder of the blood is retransfused into the donor.

Cells capable of regenerating hematopoiesis in vivo present in the isolated population of blood cells can be further purified in order to increase the concentration of said cells. Said purification may be done by positive selection of CD34 positive cells.

In a further embodiment, the invention concerns a method of preparation of a donor of circulating cells, which cells are capable of regenerating hematopoiesis in vivo comprising the administration to said donor of a composition comprising growth hormone or one of its derivatives or any factor inducing growth hormone release in an amount sufficient to increase the number of circulating cells capable of regenerating hematopoiesis in vivo in said donor.

In another embodiment, the invention concerns a method for increasing the number of circulating cells capable of regenerating hematopoiesis in vivo in a donor by administration to said donor of a composition comprising growth hormone or one of its derivatives or any factor inducing the growth hormone release.

The term "increased" or "increase" and the term "enriched" generally mean in the context of the invention that the "increased" or "enriched" parameter (number) has a value which is above the standard value of this parameter. The standard value of the parameter is measured in a body or in a sample of a body which has not received any mobilising agent of cells capable of regenerating hematopoiesis in vivo. The standard value of the number of CD34⁺ cells per microliter of blood is for example 3.8 (+ or - 3.2) cells per microliter of peripheral blood (Anderlini et al., 1997).

The circulating cells capable of regenerating hematopoiesis in vivo may be CD34⁺ cells.

The frequency of CD34⁺ cells in the blood may be measured by FACScan measurements (Siena et al., 1989 & 1991).

The increased number of CD34⁺ cells in the peripheral blood of the donor or the level of enrichment of CD34⁺ cells in the isolated preparation of blood cells may be more than 10 CD34⁺ cells per microliter of peripheral blood.

The increased number of CD34⁺ cells in the peripheral blood of the donor or the level of enrichment of CD34⁺ cells in the isolated preparation of blood cells may be at least 2x10⁶ CD34⁺ cells per kilogram of recipient body weight, or at least 4x10⁶ CD34⁺ cells per kilogram of recipient body weight or at least 8x10⁶ CD34⁺ cells per kilogram of recipient body weight.

The increased number of CD34⁺ cells in the peripheral blood of the donor or the level of enrichment of CD34⁺ cells in the isolated preparation of blood cells may be at least 2x10⁶ CD34⁺ cells per kilogram of donor body weight, at least 4x10⁶ CD34⁺ cells per kilogram of donor body weight or at least 8x10⁶ CD34⁺ cells per kilogram of donor body weight.

There is a correlation between the number of CD34⁺ cells required for transplantation and the corresponding GM-CFC activity which can be measured (Weaver et al., 1998). Therefore, the increased number of circulating cells capable of regenerating hematopoiesis in vivo or the level of enrichment of cells capable of regenerating hematopoiesis in vivo in the isolated preparation of blood cells may correspond to at least 1x10⁵ GM-CFC per kilogram of donor or recipient body weight.

The number of CD34⁺ cells in the blood correlates well with CFU-GM (Siena et al., 1991). CFU-GM is the colony forming unit, granulocyte and macrophage. Therefore, the increased number of circulating cells capable of regenerating hematopoiesis in vivo or the level of enrichment of cells capable of regenerating hematopoiesis in vivo in the isolated preparation of blood cells may correspond to at least 500 CFU-GM per milliliter of peripheral blood.

With the same reasoning, the increased number of circulating cells capable of regenerating hematopoiesis in vivo or the level of enrichment of cells capable of regenerating hematopoiesis in vivo in the isolated preparation of blood cells may correspond to an increased level of CFU-C, CFU-Meg or BFU-E. CFU-C is the colony forming unit, culture ; CFU-Meg is the colony forming unit, megakaryocyte and BFU-E is the burst forming unit, erythroid.

The number of CD34⁺ cells in the blood correlates well with the white blood cell count. Therefore, the increased number of circulating cells capable of regenerating hematopoiesis in vivo or the level of enrichment of cells capable of regenerating hematopoiesis in vivo in the isolated preparation of blood cells may correspond to at least 1000 white blood cells per microliter of peripheral blood.

The circulating cells capable of regenerating hematopoiesis in vivo may be CD34⁺/CD33⁺ cells and/or CD34⁺/CD38⁻ cells and/or CD34⁺/Thy-I cells and/or CD34⁺/Thy-I/CD38⁻ cells and/or CD33⁺ cells and/or bone-marrow stem cells and/or progenitor cells and/or long-term culture initiating cells (LTC-IC) and/or cells that fulfill self renewal potential and/or cells that fulfill pluripotential characteristics and/or cells that initiate long term bone marrow culture and/or cells that can generate multiple cell lineages. Cell lineages may be fully differentiated blood cells.

The CD34⁺/CD38⁻ cells and CD34⁺/Thy-I cells and CD34⁺/Thy-I/CD38⁻ cells are recited for example in *Anderlini et al* (see references). The CD34⁺/CD33⁺ cells and the CD33⁺ cells are recited for example in *Siena et al* ; 1991 (see references). The long-term culture initiating cells (LTC-IC) are recited for example in *Heather et al*(see references). Cells that fulfill self renewal potential and/or cells that fulfill pluripotential characteristics and/or cells that initiate long term bone marrow culture are recited for example in *Anderlini et al*(see references).

In a further embodiment, the invention relates to the following uses :
- Use of growth hormone, one of its derivatives, or any factor inducing growth hormone release to prepare a medicament for increasing or expanding the number of circulating cells capable of regenerating hematopoiesis in vivo.
- Use of growth hormone, one of its derivatives or any factor inducing growth hormone release to prepare a medicament for peripheralizing cells capable of regenerating hematopoiesis in vivo.
- Use according to any one of the preceding uses wherein the circulating cells capable of regenerating hematopoiesis in vivo are CD34⁺ cells.
- Use according to the preceding use wherein the increased number of CD34⁺ cells is more than 10 CD34⁺ cells per microliter of peripheral blood.
- Use according to any one of the preceding uses wherein the increased number of CD34⁺ cells is at least 2x10⁶ CD34⁺ cells per kilogram of recipient body weight, or at least 4x10⁶ CD34⁺ cells per kilogram of recipient body weight or at least 8x10⁶ CD34⁺ cells per kilogram of recipient body weight.
- Use according to any one of the preceding uses wherein the increased number of CD34⁺ cells is at least 2x10⁶ CD34⁺ cells per kilogram of donor body weight, at least 4x10⁶ CD34⁺ cells per kilogram of donor body weight or at least 8x10⁶ CD34⁺ cells per kilogram of donor body weight.
- Use according to any one of the preceding uses wherein the increased number of circulating cells capable of regenerating hematopoiesis in vivo corresponds to at least 1x10⁵ GM-CFC per kilogram of donor or recipient body weight.
- Use according to any one of the preceding uses wherein the increased number of circulating cells capable of regenerating hematopoiesis in vivo corresponds to at least 500 CFU-GM per milliliter of peripheral blood.
- Use according to any one of the preceding uses wherein the increased number of circulating cells capable of regenerating hematopoiesis in vivo corresponds to an increased level of CFU-C, CFU-Meg or BFU-E.
- Use according to any one of the preceding uses wherein the increased number of circulating cells capable of regenerating hematopoiesis in vivo substantially corresponds to a white blood cell count which is at least 1000 cells per microliter of peripheral blood.
- Use according to any one of the preceding uses wherein the circulating cells capable of regenerating hematopoiesis in vivo are CD34⁺/CD33⁺ cells and/or CD34⁺/CD38⁻ cells and/or CD34⁺/Thy-I cells and/or CD34⁺/Thy-I/CD38⁻ cells and/or CD33⁺ cells and/or stem cells and/or progenitor cells and/or long-term culture initiating cells (LTC-IC) and/or cells that fulfill self renewal potential and/or cells that fulfill pluripotential characteristics and/or cells that initiate long term bone marrow culture.
- Use according to any one of the preceding uses wherein the composition comprises further one or several compound(s) chosen among the following groups of compounds : hematopoietic growth factors, cytokines, chemokines, monoclonal antibodies.
- Use according to any one of the preceding uses wherein the cytokines group comprises IL-1, IL-3, G-CSF, GM-CSF or SCF ; the chemokines group comprises MIP-1α or thrombopoietin (TPO) ; the monoclonal antibodies group comprises anti-VLA-4 antibodies.
- Use according to any one of the preceding uses wherein the composition comprises Growth Hormone and G-CSF.
- Use according to any one of the two preceding uses wherein the G-CSF is administered in an amount of 10µg per kilogram.
- Use according to any one of the two preceding uses wherein the administration is made by intravenous route.
- Use according to any one of the preceding uses wherein the administration is made by parenteral, subcutaneous, intravenous, intramuscular, intraperitoneal, transdermal or buccal routes.
- Use according to any one of the preceding uses wherein the administration is daily.
- Use according to any one of the preceding uses wherein the administration is made over a period of 5 days or over a period of 10 days.
- Use according to any one of the preceding uses wherein growth hormone is recombinant growth hormone.
- Use according to any one of the preceding uses wherein growth hormone is human growth hormone.

In a second aspect, the invention concerns a method of preparation of a population of circulating cells capable of regenerating hematopoiesis in vivo comprising :
a) administering to a donor a composition comprising growth hormone or one of its derivatives or any factor inducing growth hormone release in an amount sufficient to reduce the volume of blood required to be processed in order to obtain the specified target number of circulating cells capable of regenerating hematopoiesis in vivo,
b) processing or isolating said reduced volume of blood ; and optionally
c) isolating a population of circulating cells capable of regenerating hematopoiesis in vivo from said isolated volume.

Step b) or c) [isolating a population of circulating cells capable of regenerating hematopoiesis in vivo from said isolated volume] of the methods or uses of the invention may correspond to the operation of removing peripheral blood from the donor wherein the number of cells capable of regenerating hematopoiesis in vivo has been increased by administration of growth hormone or one of its derivatives or any factor inducing growth hormone alone or in combination with other factors.

The operation of removing peripheral blood from the donor may correspond to leukapheresis. Leukapheresis is a procedure, in which, leukocytes are removed from the withdrawn blood and the remainder of the blood is retransfused into the donor.

Cells capable of regenerating hematopoiesis in vivo present in the isolated population of blood cells can be further purified in order to increase the concentration of said cells. Said purification may be done by positive selection of CD34 positive cells.

In another embodiment, the invention concerns a method of preparation of a donor of circulating cells, which cells are capable of regenerating hematopoiesis in vivo comprising the administration to said donor of a composition comprising growth hormone or one of its derivatives or any factor inducing growth hormone release in an amount sufficient to reduce the volume of blood required to be processed in order to obtain the specified target number of circulating cells capable of regenerating hematopoiesis in vivo and/or to reduce the number of leukapheresis required to collect sufficient amount of circulating cells capable of regenerating hematopoiesis in vivo to be tranplanted.

The volume of blood required to be processed may be the volume of blood required to be processed during the apheresis or leukapheresis procedure.

In a further embodiment, the invention concerns a method for reducing the volume of blood required to be processed in order to obtain the specified target number of circulating cells capable of regenerating hematopoiesis in vivo in a donor and/or for reducing the number of leukapheresis required to collect sufficient amount of circulating cells capable of regenerating hematopoiesis in vivo to be tranplanted by administration of a composition comprising the growth hormone or one of its derivatives or any factor inducing the growth hormone release to said donor .

The term "reduced" generally means in view of the invention that the "reduced" parameter (volume) has a value which is inferior to the standard value of this parameter.

The specified target number of circulating cells capable of regenerating hematopoiesis in vivo is at least 2x10⁴ LTC-IC per kg of donor or recipient body, around or more than 2x10⁶ CD34⁺ cells per kilogram of donor or recipient body weight, around or more than 4x10⁶ CD34⁺ cells per kilogram of donor or recipient body weight or around or more than 8x10⁶ CD34⁺ cells per kilogram of donor or recipient body weight.

The required volume of blood may be comprised in a range of about 30 to about 900 milliliters. In another embodiment, the invention relates to the following uses :
- Use of growth hormone, one of its derivatives or any factor inducing growth hormone release to prepare a medicament for reducing the volume of blood required to be processed in order to obtain the specified target number of circulating cells capable of regenerating hematopoiesis in vivo.
- Use according to the preceding use wherein the specified target number of circulating cells capable of regenerating hematopoiesis in vivo is around or more than 2x10⁴ LTC-IC per kg of donor or recipient body, around or more than 2x10⁶ CD34⁺ cells per kilogram of donor or recipient body weight, around or more than 4x10⁶ CD34⁺ cells per kilogram of donor or recipient body weight or around or more than 8x10⁶ CD34⁺ cells per kilogram of donor or recipient body weight.
- Use according to any one of the two preceding uses wherein the required volume of blood is comprised in a range of about 30 to about 900 milliliters.
- Use according to any one of the preceding uses wherein the composition comprises further one or several compound chosen among the following groups of compounds : hematopoietic growth factors, cytokines, chemokines, monoclonal antibodies.
- Use according to any one of the preceding uses wherein the cytokines group comprises IL-1, IL-3, G-CSF, GM-CSF or SCF ; the chemokines group comprises MIP-1α or thrombopoietin (TPO) ; the monoclonal antibodies group comprises anti-VLA-4 antibodies.
- Use according to any one of the preceding uses wherein the composition comprises human Growth Hormone and G-CSF.
- Use according to any one of the two preceding uses wherein the G-CSF is administrated in an amount of 10µg per kilogram.
- Use according to any one of the two preceding uses wherein the administration is made by intravenous route.
- Use according to any one of the preceding uses wherein the administration is made by parenteral, subcutaneous, intravenous, intramuscular, intraperitoneal, transdermal or buccal routes.
- Use according to any one of the preceding uses wherein the administration is daily.
- Use according to any one of the preceding uses wherein the administration is made over a period of 5 days or over a period of 10 days.
- Use according to any one of the preceding uses wherein the growth hormone is recombinant growth hormone.
- Use according to any one of the preceding uses wherein the growth hormone is human growth hormone.
   In this application :
- The term "circulating" may be replaced by the term "blood" or "peripheral blood".
- The term "preparation" in the expression "method of preparation" may be replaced by "pre-treament" or by "preparation for blood extraction or leukapheresis".
- A "donor" as recited in the methods or uses of the invention may be a human or an animal, a healthy or a sick individual (patient). Said animal is preferably a mammal and may be chosen from domestic animals such as dogs, cats etc. or animals such as horses, cattle, sheep.
- The term 〈〈 hematopoiesis 〉〉 can mean the formation of the blood cells.
- The term "Growth hormone" encompasses human growth hormone (hGH) and all the homologous proteins of human growth hormone of different species and all the homologs of human growth hormone in species other than human. Species other than human may be any sort of domestic animal or horse for example.

In a preferred embodiment, growth hormone is human growth hormone. Human growth hormone (hGH), also known as somatotropin is a protein hormone produced and secreted by the somatotropic cells of the anterior pituitary. hGH plays a key role in somatic growth through its effects on the metabolism of proteins, carbohydrates and lipids.

Human growth hormone is a single polypeptide chain of 101 amino acids having two disulfide bonds, one between Cys-53 and Cys-165, forming a large loop in the molecule, and the other between Cys-182 and Cys-189, forming a small loop near the C-terminus.

The term 〈〈 derivative 〉〉 in the expression 〈〈 derivatives of growth hormone 〉〉 signifies in the context of the invention, molecules which differ structurally from GH but which conserve the function of GH with respect to its direct or indirect effect on the metabolism of proteins, carbohydrates and lipids and/or its mobilisation effect and/or recovery effect (i.e. 〈〈 mobilization or peripheralisation of circulating cells capable of regenerating hematopoiesis in vivo, increase of the number of circulating cells capable of regenerating hematopoiesis in vivo, reduction of the number of leukapheresis required to collect sufficient amount of circulating cells for transplantation, reduction of the volume of blood required to be processed in order to obtain the specified target number of circulating cells capable of regenerating hematopoiesis in vivo 〉〉)

Derivatives of human growth hormone (hGH) included in the invention include naturally-occurring derivatives, variants and metabolic products, degradation products primarily of biosynthetic hGH and engineered derivatives of hGH produced by genetic methods. Any derivative of hGH can be used for the purpose of the present invention as long as it retains the biological activity of hGH in view of the invention.

Examples of derivatives are splice variants, oligomers, aggregates, proteolytic cleavage products, variants having substitutions, insertions or deletions of one or more amino acids etc.

Methionyl hGH is an example of derivative of hGH which is produced through recombinant DNA technology. This compound is actually a derivative of hGH having one additional methionine residue at its N-terminus (Goeddel et al., 1979).

Another example of derivative of hGH is a naturally occurring variant of hGH called 20-K-hGH which has been reported to occur in the pituitary as well as in the bloodstream (Lewis et al, 1978 ; Lewis et al, 1980). This compound, which lacks the 15 amino acid residues from Glu-32 to Gln-46, arises from an alternative splicing of the messenger ribonucleic acid (DeNoto et al., 1981).

Another example of derivative of hGH is acetylated at the N-terminus (Lewis et al., 1979).

Human growth hormone may further be in a monomeric, dimeric and higher molecular weight oligomeric form or in a mixture of said forms.

Human growth hormone may be in aggregated forms found both in the pituitary and in the circulation (Stolar et al., 1984 ; Stolar and Baumann, 1986).

The dimeric form of hGH may be of distinct types :
- a disulfide dimer connected through interchain disulfide bonds (Lewis et al., 1977),
- a covalent or irreversible dimer that is detected on sodium dodecylsulfate-polyacrylamide gels and that is not a disulfide dimer (Bewley and Li, 1975), and
- a non-covalent dimer which is easily dissociated into monomeric hGH by treatment with agents that disrupt hydrophobic interactions in proteins (Becker et al., 1987),
- a dimeric complex with Zn²⁺ (Cunningham et al., 1991).

Scatchard analysis has revealed that two Zn²⁺ ions associate per hGH dimer in a cooperative fashion, and this Zn²⁺-hGH dimeric complex was found to be more stable to denaturation than monomeric hGH (Cunningham et al., 1991).

A number of derivatives of hGH arise from proteolytic modifications of the molecule. The primary pathway for the metabolism of hGH involves proteolysis. The region of hGH around residues 130-150 is extremely susceptible to proteolysis, and several derivatives of hGH having nicks or deletions in this region have been described (Thorlacius-Ussing, 1987). This region is in the large loop of hGH, and cleavage of a peptide bond there results in the generation of two chains that are connected through the disulfide bond at Cys-53 and Cys-165. Many of these two-chain forms are reported to have increased biological activity (Singh et al., 1974).

Many derivatives of human growth hormone have been generated artificially through the use of enzymes. The enzymes trypsin and subtilisin, as well as others, have been used to modify hGH at various points throughout the molecule (Lewis et al., 1977). One such derivative, called two-chain anabolic protein (2-CAP), was formed through the controlled proteolysis of hGH using trypsin.

Another example of derivative of hGH is deamidated hGH. Asparagine and glutamine residues in proteins are susceptible to deamidation reactions under appropriate conditions. An example of deamidated hGH is pituitary hGH which has been shown to undergo this type of reaction, resulting in conversion of Asn-152 to aspartic acid and also, to a lesser extent, conversion of Gln-137 to glutamic acid (Lewis et al., 1981). Another example of deamidated hGH is Biosynthetic hGH which is known to degrade under certain storage conditions, resulting in deamidation at a different asparagine (Asn-149). This is the primary site of deamidation, but deamidation at Asn-152 is also seen (Becker et al., 1988). Deamidation at Gln-137 has not been reported in biosynthetic hGH.

Another example of derivative of hGH is sulfoxide hGH. Methionine residues in proteins are susceptible to oxidation, primarily to the sulfoxide. Both pituitary-derived and biosynthetic hGH undergo sulfoxidations at Met-14 and Met-125 (Becker et al., 1988). Oxidation at Met-170 has also been reported in pituitary but not biosynthetic hGH.

Another example of derivative of hGH is truncated forms of hGH which have been produced, either through the actions of enzymes or by genetic methods. 2-CAP, generated by the controlled actions of trypsin, has the first eight residues at the N-terminus of hGH removed. Other truncated versions of hGH have been produced by modifying the gene prior to expression in a suitable host. The first 13 residues have been removed to yield a derivative having distinctive biological properties in which the polypeptide chain is not cleaved (Gertler et al., 1986).

hGH and its derivatives may be produced by recombinant DNA technology which permits production of an unlimited supply of hGH in a number of different systems. Purification of hGH or its derivatives from the culture medium is facilitated by low amounts of contaminating proteins present. In fact, it has been shown that hGH can be purified on a laboratory scale by a single purification step on a reversed-phase HPLC column .

Recombinant hGH is generally marketed as vials containing hGH plus additional excipients, e.g., glycine and mannitol, in a lyophilized form. A companion diluent vial is provided, allowing the patient to reconstitute the product to the desired concentration prior to administration of the dose.

In general, no significant differences have been observed in the pharmacokinetics or biological activities of recombinant natural sequence hGH, recombinant N-methionyl-hGH, or pituitary-derived material in humans (Moore et al., 1988 ; Jorgensen et al. , 1988).

The human growth hormone as used in the present invention can include functional derivatives as noted above, as well as other types of derivatives, fragments, variants, analogs, or chemical derivatives. A functional derivative retains at least a portion of the amino acid sequence of hGH which permits its utility in accordance with the present invention, namely mobilization of circulating cells capable of regenerating hematopoiesis in vivo for example.

In the meaning of the invention, a 〈〈 derivative 〉〉 may be :
- A "fragment" of the human growth hormone according to the present invention refers to any subset of the molecule, that is, a shorter peptide.
- A "variant" of the human growth hormone according to the present invention refers to a molecule which is substantially similar to either the entire peptide or a fragment thereof. Variant peptides may be conveniently prepared by direct chemical synthesis of the variant peptide, using methods well known in the art.

Alternatively, amino acid variants of hGH can be prepared by mutations in the cDNA encoding the synthetized hGH derivatives. Such variants comprise deletions, insertions or substitution of residues within the amino acid sequence. Any combination of deletions, insertions, and subtitutions may also be made, provided that the final construct possesses the desired activity.

At the genetic level, these variants ordinarily are prepared by site-directed mutageneis (as exemplified by (Adelman et al., 1983)) of nucleotides in the DNA encoding the peptide molecule, thereby producing DNA encoding the variant, and thereafter expressing the DNA in recombinant cell culture. The variants typically exhibit the same biological activity as the non-variant peptide.
- An "analog" of human growth hormone according to the present invention refers to a non-natural molecule which is substantially similar to either the entire molecule or to an active fragment thereof.
- A "chemical derivative" of human growth hormone according to the present invention contains additional chemical moieties not normally part of the human growth hormone derivative amino acid sequence. Covalent modifications of the amino acid sequence are included within the scope of this invention. Such modifications may be introduced into the human growth hormone by reacting targeted amino acid residues of the peptide with an organic derivatizing agent that is capable of reacting with selected side chains or terminal residues.

The types of substitutions which may be made in the human growth hormone according to the present invention may be based on analysis of the frequencies of amino acid changes between a homologous protein of different species. Based upon such analysis, conservative substitutions may be defined herein as exchanges within one of the following five groups :
I : Small, aliphatic, nonpolar or slightly polar residues : Ala, Ser, Thr, Pro, Gly
II : Polar, negatively-charged residues and their amides : Asp, Asn, Glu, Gln
III : Polar, positively-charged residues : His, Arg, Lys
IV : Large, aliphatic non-polar residues : Met, Leu, Ile, Val, Cys
V : Large aromatic residues : Phe, Try, Trp

Within the foregoing groups, the following substitutions are considered to be "highly conservative" :
- Asp/Glu
- His/Arg/Lys
- Phe/Tyr/Trp
- Met/Leu/Ile/Val

Semi-conservative substitutions are defined to be exchanges between two of groups (I)-(IV) above which are limited to supergroup (A), comprising (I), (II), and (III) above, or to supergroup (B), comprising (IV) and (V) above. Substitutions are not limited to the genetically encoded or even the naturally- occurring amino acids. When the epitope is prepared by peptide synthesis, the desired amino acid may be used directly. Alternatively, a genetically encoded amino acid may be modified by reacting it with an organic derivatizing agent that is capable of reacting with selected side chains or terminal residues.

Cysteinyl residues most commonly are reacted with alpha- haloacetates (and corresponding amines), such as chloroacetic acid or chloroacetamide, to give carboxylmethyl or carboxyamidomethyl derivatives. Cysteinyl residues also are derivatized by reaction with bromotrifluoroacetone, alpha-bromo-beta-(5-imidazoyl)propionic acid, chloroacetyl phosphate, N-alkylmaleimides, 3-nitro-2-pyridyl disulfide, methyl-2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylprocarbonate at pH 5.5-7.0 because this agent is relatively specific for the histidyl side chain. Parabromophenacyl bromide is also useful ; the reaction is preferably performed in 0.1 M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing alpha-amino acid-containing residues include imidoesters such as methyl picolinimidate ; pyridoxal phosphate ; pyridoxal ; chloroborohydride; trinitrobenzenesulfonic acid ; O-methyliosurea ; 2,4-pentanedione ; and transaminase-catalyzed reaction with glyoxylate.

Arginyl residues are modified by reaction with one or several conventional reagents, among them phenylglyoxal ; 2,3- butanedione ; and ninhydrin. Derivatization of arginine residues requires that the reaction be performed in alkaline conditions because of the high pKa of the guanidine functional group. Furthermore, these reagents may react with the groups of lysine, as well as the arginine epsilon-amino group.

The specific modification of tyrosyl residues per se has been studied extensively, with particular interest in introducing spectral labels into tyrosyl residues by reaction with aromatic diazonium compounds or tetranitromethane. Most commonly, N-acetylimidazole and tetranitromethane are used to form O-acetyl tyrosyl species and e-nitro derivatives, respectively.

Carboxyl side groups (aspartyl or glutamyl) are selectively modified by reaction with carbodiimides (R'N-C-N-R') such as 1-cyclohexyl-3-[2-morpholinyl-(4-ethyl)]carbodiimide or 1-ethyl-3-(4-azonia-4,4-dimethylpentyl)carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues. Alternatively, these residues are deamidated under mildly acidic conditions. Either form of these residues falls within the scope of this invention.

While the present invention may be carried out with recombinant human growth hormone derivatives made by recombinant DNA technology, for instance in procaryotic or eucaryotic cells, these derivatives can also be made by conventional protein synthesis methods which are well known to those skilled in the art.

Growth hormone may be a protein, a peptide, a DNA molecule, a RNA molecule. DNA molecule and RNA molecule may encode hGH and all its derivatives including those recited above.

Growth hormone may preferably be recombinant growth hormone.

Determination of amounts of growth hormone, of one of its derivatives or of any factor inducing growth hormone release one of its derivatives or of any factor inducing growth hormone release to be administered is within the skill of the art.

Typical dosage of growth hormone, of one of its derivatives or of any factor inducing growth hormone release will start at about 1 microgram per kilogram of the patient weight per day and dose will be escalated until the desired effect (mobilization or peripheralisation of circulating cells capable of regenerating hematopoiesis in vivo, increase of the number of circulating cells capable of regenerating hematopoiesis in vivo, reduction of the number of leukapheresis required to collect sufficient amount of circulating cells for transplantation, reduction of the volume of blood required to be processed in order to obtain the specified target number of circulating cells capable of regenerating hematopoiesis in vivo) is reached.

The dosage of growth hormone, of one of its derivatives or of any factor inducing growth hormone release administered depends upon the age, sex, health and weight of the donor, type of previous or concurrent treatment, if any, frequency of the treatment and the nature of the effect desired.

The total dose required for each treatment may be administered in multiple or single dose.

Growth hormone may be administered alone or in conjunction with other factors.

Growth hormone or its derivatives or any factor inducing growth hormone release may advantageously be present in a composition which comprises further one or several compound(s) chosen among the compounds belonging to the following groups : hematopoietic growth factors, cytokines, chemokines, monoclonal antibodies.

The cytokine group can comprise IL-1,IL-3, G-CSF, GM-CSF or SCF. The chemokine group can comprise MIP-1α or thrombopoietin (TPO). The monoclonal antibody group can comprise anti-VLA-4 antibodies.

Growth hormone or its derivatives or any factor inducing growth hormone release may advantageously be present in a composition which comprises granulocyte colony stimulating factor (G-CSF).

The G-CSF may be administered in an amount of 10µg per kilogram of body weight.

The pharmaceutical compositions or compositions which are used in the methods and uses of the invention can comprise growth hormone or one of its derivatives or any factor inducing growth hormone release in a pharmaceutical acceptable form optionally combined with an acceptable carrier.

These compositions can be administered by any means that achieve their intended purposes.

The composition comprising growth hormone or its derivatives or any factor inducing growth hormone release may be administered alone or in conjunction with other therapeutics directed to a disease or directed to other symptoms thereof.

The composition comprising growth hormone or one of its derivatives or any factor inducing growth hormone release may be administered by the intravenous route.

After intravenous administration, the elimination of hGH is described by first-order kinetics with a serum half-life of 12- 30 minutes in both animals and humans (Moore et al., 1988; Hendricks et al., 1985). Traditionally, intramuscular injection has been the method of choice as the preferred route of delivery. In humans, absorption of exogenous hGH appears to be more rapid from the intramuscular site, with a time to maximum concentration of two to three hours, compared to four to six hours after subcutaneous administration. The disappearance phase from serum has been reported to range from 12-20 hours for intramuscular administration, and 20-24 hours after subcutaneous administration (Albertsson-Wikland et al., 1986; Jorgensen et al., 1987).

The composition comprising growth hormone or one of its derivatives or any factor inducing growth hormone release may be administered by parenteral routes such as subcutaneous, intravenous, intramuscular, intraperitoneal, or transdermal route or by mucosal routes such as buccal or oral route.

The composition comprising growth hormone or one of its derivatives or any factor inducing growth hormone release may be administered daily.

The composition comprising growth hormone or one of its derivatives or any factor inducing growth hormone release may be a daily administration that can start up to 20 days pre-leukapheresis.

The composition comprising growth hormone or one of its derivatives or any factor inducing growth hormone release may be administered over a period of 5 days or over a period of 10 days.

Methods and uses of the invention may be combined with a prior treatment called 〈〈 chemopriming 〉〉. The 〈〈 chemopriming 〉〉 regimens which may be used are :
- high-dose cyclophosphamide (4 g/m²) for patients with breast cancer or multiple myeloma,
- ifosfamide, etoposide for patients with non-Hodgkin's lymphoma or Hodgkin's disease,
- cyclophosphamide, etoposide, cisplatin (CVP) for patients with solid tumors (e.g., breast cancer).

To enhance the induction of a cells capable of regenerating hematopoiesis in vivo rebound, methods and uses of the invention are started shortly after completion of chemopriming treatment and continued until completion of apheresis (5 to 12 µg/kg/d).

It is also noteworthy that in patients whose marrow stem cell pool is significantly diminished by prior chemotherapy, an additional chemopriming regimen might impair rather than induce cells capable of regenerating hematopoiesis in vivo peripheralization. Stem cells toxic chemotherapeutic agents such as busulfan, doxorubucin, melphalan, thiotepa and possibly fludarabine (and others) should not be part of a chemopriming regimen. On the other hand, cyclophosphamide is considered the ideal chemopriming drug with the least cells capable of regenerating hematopoiesis in vivo toxicity, although cardiotoxicity (dose > 4g/m²) and hemorrhagic cystitis are the well-known dose-limiting extramedullary side effects (Shepperd et al., 1990).

The population of blood cells enriched with cells capable of regenerating hematopoiesis in vivo obtained from the peripheral blood by the methods and uses of the invention can be re-infused, grafted or transplanted into the same individual which is in this case the donor (autologous transplantation) or into different individuals (nonautologous transplantation).

Said operation of re-infusion, engraftment or transplantation belongs to the so-called Hematopoietic Stem Cells Transplantation (HSCT) procedures. HSCT is a clinical procedure in which cells capable of regenerating hematopoiesis in vivo, obtained from bone marrow or peripheral blood, are transplanted to a patient

An autologous transplantation is a transplantation in which donor and recipient are the same individual whereas a nonautologous transplantation is a transplantation in which donor and recipient are different individuals. The method of the invention encompasses both autologous and non-autologous transplantation.

In a second part, the invention provides new uses for enhancing hematopoietic reconstitution. The invention relates to an agent capable of promoting, enhancing or accelerating the hematopoietic regeneration, recovery or reconstitution. The invention provides new uses for enhancing hematopoietic reconstitution.

Thus, the invention relates to the use of human growth hormone or one of its derivatives or any factor inducing human growth hormone release to prepare a medicament for enhancing hematopoietic reconstitution, in a human being.

Throughout the application, the term 〈〈 enhancing 〉〉 and all terms having the same root may be replaced by the term 〈〈 promoting 〉〉 or by the term 〈〈 accelerating 〉〉.

Throughout the application, the term 〈〈 reconstitution 〉〉 and all terms having the same root may be replaced by the term 〈〈 recovery 〉〉 or by the term 〈〈 regeneration 〉〉.

In another embodiment, the invention relates to the use of human growth hormone or one of its derivatives or any factor inducing human growth hormone release to prepare a medicament for enhancing hematopoietic reconstitution following bone marrow transplantation in a human being.

In another embodiment, the invention relates to the use of human growth hormone or one of its derivatives or any factor inducing human growth hormone release to prepare a medicament for enhancing engraftment of bone marrow or cells capable of regenerating hematopoiesis in vivo in a human being.

Another embodiment of the invention is the use of growth hormone or one of its derivatives or any factor inducing growth hormone release to prepare a medicament for enhancing hematopoietic reconstitution after transplantation of cells capable of regenerating hematopoiesis in vivo.

In a further embodiment, the invention relates to the use of growth hormone or one of its derivatives or any factor inducing growth hormone release to prepare a medicament for enhancing engraftment of cells capable of regenerating hematopoiesis in vivo.

Growth hormone may advantageously be human growth hormone.

Growth hormone and its derivatives may correspond to growth hormone and its derivatives which are recited above in this application in connection with the first part of the invention.

The hematopoietic reconstitution or the enhanced engraftment may be detected by an increase of the peripheral White Blood Cell (WBC) count and/or granulocytes count and/or lymphocytes count and/or platelet count and/or erythrocyte count.

An increase of the peripheral White Blood Cell (WBC) count and/or granulocytes count and/or lymphocytes count and/or platelet count and/or erythrocyte count may be detected by comparison with the rate of increase of said counts in an individual who has received the same transplantation regimen but who has not received any hematopoietic reconstitution treatment.

The hematopoietic reconstitution or the enhanced engraftment may be detected by reduction of the period of time necessary to recover a normal or standard peripheral White Blood Cell (WBC) count and/or granulocytes count and/or neutrophil count and/or lymphocytes count and/or platelet count and/or erythrocyte count.

A normal or standard peripheral White Blood Cell (WBC) count and/or granulocytes count and/or neutrophil count and/or lymphocytes count and/or platelet count and/or erythrocyte count is the one which is measured in a healthy individual or in an individual which has not received any myeloablative or myelosuppressive therapy or any transplantation.

A normal neutrophil count may be at least 0.5x10⁹ neutrophil cells per liter of peripheral blood.

A normal platelet count may be at least 20x10⁹ per liter of peripheral blood.

The hematopoietic reconstitution or enhancing engrafment may be detected by a reduction of the extent and/or duration of neutropenia and/or thrombocytopenia and/or anemia and/or hemorrhages and/or duration of prophylaxis.

The hematopoietic reconstitution or the enhancing engrafment may be detected by a reduction of the duration and/or severity of fever and/or infections.

A reduction of the extent and/or duration of neutropenia and/or thrombocytopenia and/or anemia and/or hemorrhages and/or duration of prophylaxis or a reduction of the duration and/or severity of fever and/or infections may be compared with said extent and/or duration and/or severity measured in an individual who has received the same transplantation regimen but who has not received any hematopoietic reconstitution treatment.

The hematopoietic reconstitution or the enhancing engrafment may be detected by a recovery of granulocytes which is at least 1000 per microliter of peripheral blood.

The hematopoietic reconstitution or the enhancing engrafment may be detected by a recovery of platelet count which is at least 50,000 per microliter of peripheral blood.

In another embodiment, the invention relates to the use of growth hormone or one of its derivatives or any factor inducing growth hormone release to prepare a medicament for treating a neoplastic disease, an hematological disorder, malignancies, Severe Combined Immune Deficiencies (SCIDs), congenitally or genetically determined hematopoietic abnormalities, anemia, aplastic anemia, leukemia and/or osteopetrosis. A neoplastic disease may be breast cancer.

In another embodiment, the invention relates to the use of growth hormone or one of its derivatives or any factor inducing growth hormone release to prepare a medicament for reducing the bone marrow aplasia period which follows transplantation, for preventing or treating opportunistic infections after transplantation or for limiting the risk of tumor recurrence after transplantation.

In a further embodiment, the invention relates to the use of growth hormone or one of its derivatives or any factor inducing growth hormone release to prepare a medicament for preventing or treating secondary effects of myelosuppressive therapy and/or radiotherapy and/or chemotherapy.

In a further embodiment, the invention relates to the use of growth hormone or one of its derivatives or any factor inducing growth hormone release to prepare a medicament for preventing or treating neutropenia and/or thrombocytopenia.

In a further embodiment, the invention relates to the use of growth hormone or one of its derivatives or any factor inducing growth hormone release to prepare a medicament for preventing or treating anemia following radiotherapy and/or chemotherapy and /or hematopoietic stem cells transplantation and/or transplantation of cells capable of regenerating hematopoiesis and/or Bone marrow transplantation.

In a further embodiment, the invention relates to the use of growth hormone or one of its derivatives or any factor inducing growth hormone release to prepare a medicament for preventing or treating neutropenia, following radiotherapy and/or chemotherapy and /or hematopoietic stem cells transplantation and/or transplantation of cells capable of regenerating hematopoiesis and/or Bone marrow transplantation.

In a further embodiment, the invention relates to the use of growth hormone or one of its derivatives or any factor inducing growth hormone release to prepare a medicament for preventing or treating thrombocytopenia, following radiotherapy and/or chemotherapy and /or hematopoietic stem cells transplantation and/or transplantation of cells capable of regenerating hematopoiesis and/or Bone marrow transplantation.

The cells capable of regenerating hematopoiesis in vivo may belong to one or several of the following groups of cells : CD34⁺ cells, CD34⁺CD33⁺ cells, CD34⁺CD38⁻ cells, CD34⁺Thy-I cells, CD34⁺Thy-ICD38⁻cells, CD33⁺ cells, stem cells, progenitor cells, long-term culture initiating cells (LTC-IC), cells that fulfill self renewal potential, cells that fulfill pluripotential characteristics, cells that initiate long term bone marrow culture.

Determination of amounts of growth hormone, of one of its derivatives or of any factor inducing growth hormone release one of its derivatives or of any factor inducing growth hormone release to be administered is within the skill of the art.

Typical dosage of growth hormone, of one of its derivatives or of any factor inducing growth hormone release will start at about 1 micrograms per kilogram of the patient weight per day and dose will be escalated until the desired effect (mobilization or peripheralisation of circulating cells capable of regenerating hematopoiesis in vivo, increase in said donor of the number of circulating cells capable of regenerating hematopoiesis in vivo, reduction of the number of leukapheresis required to collect sufficient amount of circulating cells for transplantation, reduction of the volume of blood required to be processed in order to obtain the specified target number of circulating cells capable of regenerating hematopoiesis in vivo) is reached.

The dosage of growth hormone, of one of its derivatives or of any factor inducing growth hormone release administered depends upon the age, sex, health and weight of the donor, type of previous or concurrent treatment, if any, frequency of the treatment and the nature of the effect desired.

The total dose required for each treatment may be administered in multiple or single dose.

The medicament may comprise further one or several compound(s) chosen among the following groups of compounds : hematopoietic growth factors, cytokines, chemokines, monoclonal antibodies.

The cytokines group comprises IL-1,IL-3, IL-6, IL-11, Insulin-like growth factor 1 (IGF-1), G-CSF, GM-CSF or SCF. The chemokines group comprises MIP-1α and thrombopoietin (TPO). The monoclonal antibodies group comprises anti-VLA-4 antibodies.

The medicament may comprise Growth Hormone and G-CSF.

G-CSF may be administered in an amount of 10µg per kilogram of body weight.

The administration of the medicament may be made by intravenous route.

The administration of the medicament may be made by parenteral routes such as the subcutaneous, intravenous, intramuscular, intraperitoneal, transdermal route or by mucosal routes such as the buccal or oral route.

The administration of the medicament may be made daily.

The administration of the medicament may be made over a period of 3 days along.

The administration of the medicament may be made from day 1 to day 3 after transplantation.

The term 〈〈 transplantation 〉〉 encompasses bone marrow transplantation or hematopoietic stem cells transplantation.

Growth hormone used in the medicament may advantageously be recombinant growth hormone.

Growth hormone used in the medicament may advantageously be human growth hormone.

In a third part, the invention provides a combination of the methods and uses of the first part of the invention (mobilisation) with the methods and uses of the second part of the invention (recovery).

Said combination methods and uses are mobilisation and recovery methods which may be applied in cases autologous transplantation of hematopoietic stem cells wherein the donor and the recipient is the same person or individual. Thus, growth hormone or one of its derivatives or any factor inducing growth hormone release can be used as a mobilising agent in a first mobilisation step which is a pre-treatment in view of blood cells extraction and as an hematopoietic recovery agent in a second step following transplantation.

Said combination methods and uses are very useful. In fact, transplantation of cells mobilized by growth hormone or one of its derivatives or any factor inducing growth hormone release to a patient results in faster haematological recovery than transplantation without a prior mobilization treatment of said patient.

All methods and uses of the invention can be applied in at least three clinically important fields, namely autologous bone marrow transplantation, allogeneic bone marrow transplantation, and gene therapy.

### Examples

### Abbreviations and notes :

- - **BFU-E** :: burst forming unit, erythroid
- - **CFU-C :**: colony forming unit, culture
- - **CFU-GM** :: colony forming unit, granulocyte and macrophage
- - **CFU-Meg** :: colony forming unit, megakaryocyte
- - **G-CSF :**: granulocyte colony stimulating factor
- - **IGF-I :**: insulin growth factor I
- - **LTC-IC :**: long term culture initiating cell
- - **HGH :**: human growth hormone
- - **RhG-CSF :**: recombinant human granulocyte colony stimulating factor
- - **RhGH :**: recombinant human growth hormone

### Example 1 : Mobilization activity of hGH studied in a murine preclinical model

BALB/c mice are given 10µL intraperitoneal injections of rhGH every day for total of 10 days. The total CFU-C or BFU-E activity circulating in the peripheral blood on day 5 and day 10, respectively, is determined according to standard in vitro culture techniques, and compared with :
(i) steady-state pretreatment levels,
(ii) absolute CFU-C and BFU-E counts on day 3 and day 5, respectively following treatment with rhG-CSF given intraperitoneally at 10µL every day for 5 consecutive days.

### Example 2 : Selection criteria for the mobilization and recovery clinical studies

### A) Inclusion criteria :

- Written informed consent
- Age 18 years and 60 years
- Histologically confirmed high-risk cancer (lymphome cancer) undergoing high-dose chemotherapy according to current INT guidelines.

### B) Exclusion criteria :

- Patients heavily pretreated with chemotherapy courses of chemotherapy) and/or radiotherapy.
- Renal (creatinine > 1,5 N), or hepatic insufficience and/or SGPT > 2,5 N ; bilirubin > 1,5 N), or severe CNS or psychiatric disease.
- Clinically significant cardiac disease or myocardiac. Left ventricular ejection fraction < 50% at rest by echocardiography assessment or < 55% by isotopic measurement.
- Hepatitis B or C, or HIV test positive.

### Example 3 : Baseline study procedure for the mobilization and recovery clinical studies

Several parameters are studied during the mobilisation and recovery clinical studies :
- Complete medical history, physical examination, cardiac examination, left ventricular ejection fraction (LVEF) by multigated scintigraphic scan or echography, chest X-ray
- Pregnancy test (if applicable)
- HBV, HCV and HIV test
- Complete blood count with differential
- Absolute counts of circulating CD34⁺ cells and CFU
- Blood chemistry (transaminases, serum phosphatase, gammaGT, LDH, total bilirubin, BUN, creatinine, glycemia, Na, K, Ca, P, uric acid, total protein, albumin, cholesterol, triglycerides
- Bilateral bone marrow biopsy
- Informed consent

### Example 4 : Main parameters of toxicity for the mobilization and recovery clinical studies

- Tumor growth (mobilization study only)
- Clinical and instrumental symptoms
- Laboratory tests for cardiac, liver and renal function

### Example 5 : Mobilization clinical study

### A) Objectives of the mobilization clinical study

- To assess the activity of rhGH in :
   (i) increasing circulating CD34⁺ cells, and
   (ii) expanding the bone marrow hematopoietic compartment, so to allow an enhanced mobilization by subsequent rhG-CSF administration
- To assess the safety and tolerability of rhGH, given with rhG-CSF to cancer patients following chemotherapy (hematologic recovery study).

### B) Treatment plan

### Mobilization study with rhGH :

- rhGH is administered from day 1 to 10 by the intravenous route. Dosage of rhGH is started at about 1 micrograms per kilogram of the patient weight per day and dose will be escalated until the desired effect (mobilization or peripheralisation of circulating cells capable of regenerating hematopoiesis in vivo, increase in said donor of the number of circulating cells capable of regenerating hematopoiesis in vivo, reduction of the number of leukapheresis required to collect sufficient amount of circulating cells for transplantation, reduction of the volume of blood required to be processed in order to obtain the specified target number of circulating cells capable of regenerating hematopoiesis in vivo) is reached.xx µg/kg QD, iv)

### Mobilization study with rhGH and rhG-CSF :

- rhGH administration : rhGH is administered from day 1 to 5 by the intravenous route. Dosage of rhGH is started at about 1 micrograms per kilogram of the patient weight per day and dose will be escalated until the desired effect (mobilization or peripheralisation of circulating cells capable of regenerating hematopoiesis in vivo, increase in said donor of the number of circulating cells capable of regenerating hematopoiesis in vivo, reduction of the number of leukapheresis required to collect sufficient amount of circulating cells for transplantation, reduction of the volume of blood required to be processed in order to obtain the specified target number of circulating cells capable of regenerating hematopoiesis in vivo) is reached.
- rhG-CSF administration (10 µg/kg QD, iv) from completion of CD34⁺ cell harvest (target cell dose is 8 X 10e⁶ CD34⁺ cells/kg body weight).

### C) Main parameters of activity

Starting from day +6, the following parameters are assessed :
- Absolute CD34⁺ cell counts/µL (daily in the periphery and once in the leukapheresed cells)
- Absolute CFU-GM counts/µL (daily in the periphery and once in the leukapheresed cells)

### D) Study procedure

- Daily assessment of CD34⁺ cells/µL and CFU-GM peripheral blood, from day +5 until leukapheresis.
- Total yield of CD34⁺ cells, CFU-GM, BFU-E, CFU IC in leukapheresed cells.
- Toxicity assess through clinical and in examinations (EKG, chest X-ray, and other examinations as required).
- Measurement and evaluation of all tumor parameters ending the mobilization study

### Example 6 : Recovery clinical study

### A) Objectives of the recovery clinical study

- To assess the ability of rhGH, given alone or in combination to hasten the recovery of WBC, RBC and platelets in the peripheral blood of cancer patients treated with high-dose chemotherapy and peripheral blood stem-cell autografting.
- To assess the safety and tolerability of rhGH, given with rhG-CSF to cancer patients following chemotherapy.

### B) Treatment plan

- Administration of high-dose chemotherapy, followed by infusion on day 0 of an optimal amount (i.e. 8X10e⁶ CD34⁺ cells/kg) of crypreserved cells harvested in the mobilization study.
- Co-administration (iv) of rhGH and rhG-CSF µg/kg QD, iv) from day 1 until stable (i.e. for three consecutive days) recovery of granulocytes above 1000µL, and platelet counts above 50,000/µL.

### C) Main parameters of activity

Starting from day +0, and until full and stable recovery, the following parameters will be assessed :
- Absolute granulocyte counts/µL (daily)
- Absolute platelet counts/µL (daily)
- Absolute erythrocyte counts/µL (daily)
- Granulocyte nadir
- Platelet nadir
- Extent and duration of neutropenia
- Extent and duration of thrombocytopenia
- Extent and duration of hematopoietic support tranfusions, RBC transfusions)
- Duration of infectious prophylaxis, and infections
- Hemorrhages

### D) Study procedure

- Daily assessment of WBC, RBC and platelet count
- Number of platelet transfusions
- Number of RBC transfusions
- Type and severity of fever and documented infection
- Clinical and instrumental assessment of toxicities hematological

### References

Adelman, J.P., Hayflick, J.S., Vasser, M., and Seeburg, P.H. (1983). In vitro deletional mutagenesis for bacterial production of the 20,000- dalton form of human pituitary growth hormone. DNA 2, 183-193.
Albertsson-Wikland, K., Westphal, O., and Westgren, U. (1986). Daily subcutaneous administration of human growth hormone in growth hormone deficient children. Acta Paediatr.Scand. 75, 89-97.
Alexander, W.S. (1998). Cytokines in hematopoiesis. Int.Rev.Immunol. 16, 651-682.
Anderlini, P. and Korbling, M. (1997). The use of mobilized peripheral blood stem cells from normal donors for allografting. Stem.Cells 15, 9-17.
Becker, G.W., Bowsher, R.R., Mackellar, W.C., Poor, M.L., Tackitt, P.M., and Riggin, R.M. (1987). Chemical, physical, and biological characterization of a dimeric form of biosynthetic human growth hormone. Biotechnol.Appl.Biochem. 9, 478-487.
Becker, G.W., Tackitt, P.M., Bromer, W.W., Lefeber, D.S., and Riggin, R.M. (1988). Isolation and characterization of a sulfoxide and a desamido derivative of biosynthetic human growth hormone. Biotechnol.Appl.Biochem. 10, 326-337.
Bewley, T.A. and Li, C.H. (1975).
   The chemistry of human pituitary growth hormone. Adv.Enzymol.Relat.Areas.Mol.Biol. 42:73-166, 73-166.
Cunningham, B.C., Mulkerrin, M.G., and Wells, J.A. (1991). Dimerization of human growth hormone by zinc. Science 253, 545-548.
DeNoto, F.M., Moore, D.D., and Goodman, H.M. (1981). Human growth hormone DNA sequence and mRNA structure : possible alternative splicing. Nucleic.Acids.Res. 9, 3719-3730.
Derfalvi, B., Sallai, P., Nemet, K., Szalai, C., Kenesei, E., Tulassay, T., and Falus, A. (1998). [The in vitro effect of recombinant human growth hormone on lymphocyte and granulocyte function in healthy and uremic children]. Orv.Hetil. 139, 1847-1850.
Fischer, A., Haddad, E., Jabado, N., Casanova, J.L., Blanche, S., Le Deist, F., and Cavazzana-Calvo, M. (1998). Stem cell transplantation for immunodeficiency. Springer Semin.Immunopathol. 19, 479-492.
Geffner, M. (1997). Effects of growth hormone and insulin-like growth factor I on T- and B- lymphocytes and immune function. Acta Paediatr.Suppl. 423 :76-9, 76-79.
Gertler, A., Shamay, A., Cohen, N., Ashkenazi, A., Friesen, H.G., Levanon, A., Gorecki, M., Aviv, H., Hadary, D., and Vogel, T. (1986). Inhibition of lactogenic activities of ovine prolactin and human growth hormone (hGH) by a novel form of a modified recombinant hGH. Endocrinology 118, 720-726.
Gianni, A.M., Bregni, M., Siena, S., Villa, S., Sciorelli, G.A., Ravagnani, F., Pellegris, G., and Bonadonna, G. (1989). Rapid and complete hemopoietic reconstitution following combined transplantation of autologous blood and bone marrow cells. A changing role for high dose chemo-radiotherapy ? Hematol.Oncol. 7, 139-148.
Goeddel, D.V., Heyneker, H.L., Hozumi, T., Arentzen, R., Itakura, K., Yansura, D.G., Ross, M.J., Miozzari, G., Crea, R., and Seeburg, P.H. (1979). Direct expression in Escherichia coli of a DNA sequence coding for human growth hormone. Nature 281, 544-548.
Heather J. et al. Blood 74 ; 1563-1570 (1989).
Hendricks, C.M., Eastman, R.C., Takeda, S., Asakawa, K., and Gorden, P. (1985). Plasma clearance of intravenously administered pituitary human growth hormone: gel filtration studies of heterogeneous components. J.Clin.Endocrinol.Metab. 60, 864-867.
Henry, D. (1997). Haematological toxicities associated with dose-intensive chemotherapy, the role for and use of recombinant growth factors. Ann.Oncol. 8 Suppl 3:S7-10, S7-10.
Jorgensen, J.O., Flyvbjerg, A., Dinesen, J., Lund, H., Alberti, K.G., Orskov, H., and Christiansen, J.S. (1987). Serum profiles and short-term metabolic effect of pituitary and authentic biosynthetic human growth hormone in man. A double-blind cross-over study. Acta Endocrinol.(Copenh.) 116, 381-386.
Jorgensen, K.D., Monrad, J.D., Brondum, L., and Dinesen, B. (1988). Pharmacokinetics of biosynthetic and pituitary human growth hormones in rats. Pharmacol.Toxicol. 63, 129-134.
Kimata, H. and Yoshida, A. (1994). Effect of growth hormone and insulin-like growth factor-I on immunoglobulin production by and growth of human B cells. J.Clin.Endocrinol.Metab. 78, 635-641.
Larsson, K., Bjorkstrand, B., and Ljungman, P. (1998). Faster engraftment but no reduction in infectious complications after peripheral blood stem cell transplantation compared to autologous bone marrow transplantation. Support.Care Cancer 6, 378-383.
Lewis, U.J., Peterson, S.M., Bonewald, L.F., Seavey, B.K., and VanderLaan, W.P. (1977). An interchain disulfide dimer of human growth hormone. J.Biol.Chem. 252, 3697-3702.
Lewis, U.J., Singh, R.N., Bonewald, L.F., Lewis, L.J., and VanderLaan, W.P. (1979). Human growth hormone: additional members of the complex. Endocrinology 104, 1256-1265.
Lewis, U.J., Singh, R.N., Bonewald, L.F., and Seavey, B.K. (1981). Altered proteolytic cleavage of human growth hormone as a result of deamidation. J.Biol.Chem. 256, 11645-11650.
Lewis, U.J., Singh, R.N., VanderLaan, W.P., and Tutwiler, G.F. (1977). Enhancement of the hyperglycemic activity of human growth hormone by enzymic modification. Endocrinology 101, 1587-1603.
Merchav, S., Tatarsky, I., and Hochberg, Z. (1988). Enhancement of erythropoiesis in vitro by human growth hormone is mediated by insulin-like growth factor I. Br.J.Haematol. 70, 267-271.
Moore, J.A., Rudman, C.G., MacLachlan, N.J., Fuller, G.B., Burnett, B., and Frane, J.W. (1988). Equivalent potency and pharmacokinetics of recombinant human growth hormones with or without an N-terminal methionine. Endocrinology 122, 2920-2926.
Siena et al. Blood 74 ; 1905-1914 (1989)
Siena et al. Blood 77 ; 400-409 (1991)
Singh, R.N., Seavey, B.K., Rice, V.P., Lindsey, T.T., and Lewis, U.J. (1974). Modified forms of human growth hormone with increased biological activities. Endocrinology 94, 883-891.
Stolar, M.W., Amburn, K., and Baumann, G. (1984). Plasma "big" and "big-big" growth hormone (GH) in man : an oligomeric series composed of structurally diverse GH monomers. J.Clin.Endocrinol.Metab. 59, 212-218.
Stolar, M.W. and Baumann, G. (1986). Big growth hormone forms in human plasma : immunochemical evidence for their pituitary origin. Metabolism 35, 75-77.
Thorlacius-Ussing, O. (1987). Zinc in the anterior pituitary of rat : a histochemical and analytical work. Neuroendocrinology. 45, 233-242.
Tian, Z.G., Woody, M.A., Sun, R., Welniak, L.A., Raziuddin, A., Funakoshi, S., Tsarfaty, G., Longo, D.L., and Murphy, W.J. (1998). Recombinant human growth hormone promotes hematopoietic reconstitution after syngeneic bone marrow transplantation in mice. Stem.Cells 16, 193-199.
To LB, Shepperd KM, Haylock DN et al. Single high doses of cyclophosphamide enable the collection of high numbers of hemopoietic stem cells from the peripheral blood. Exp Hematol (1990) ; 18, 442-447.
Valerio, G., Di Maio, S., Salerno, M., Argenziano, A., Badolato, R., and Tenore, A. (1997). Assessment of red blood cell indices in growth-hormone-treated children. Horm.Res. 47, 62-66.
Van Hoef, M.E. (1998). Haematological recovery after high-dose consolidation chemotherapy with peripheral blood progenitor cell rescue: the effects of the mobilization regimen and post-transplant growth factors. Neth.J.Med. 52, 30-39.
Vihervuori, E., Virtanen, M., Koistinen, H., Koistinen, R., Seppala, M., and Siimes, M.A. (1996). Hemoglobin level is linked to growth hormone-dependent proteins in short children. Blood 87, 2075-2081.
Waters, T.M., Bennett, C.L., Pajeau, T.S., Sobocinski, K.A., Klein, J.P., Rowlings, P.A., and Horowitz, M.M. (1998). Economic analyses of bone marrow and blood stem cell transplantation for leukemias and lymphoma : what do we know ? Bone Marrow Transplant. 21, 641-650.
Weaver, A. and Testa, N.G. (1998). Stem cell factor leads to reduced blood processing during apheresis or the use of whole blood aliquots to support dose-intensive chemotherapy. Bone Marrow Transplant. 22, 33-38.

## Claims

1. Method of preparation of a population of circulating cells capable of regenerating hematopoiesis in vivo comprising :
a) administering to a donor a composition comprising growth hormone or one of its derivatives or any factor inducing growth hormone release in an amount sufficient to increase in said donor the number of circulating cells capable of regenerating hematopoiesis in vivo.
b) isolating a population of circulating cells capable of regenerating hematopoiesis in vivo from the peripheral blood of said donor.

2. Method of preparation of a donor of circulating cells, which cells are capable of regenerating hematopoiesis in vivo comprising the administration to said donor of a composition comprising growth hormone or one of its derivatives or any factor inducing the growth hormone release in an amount sufficient to the number of circulating cells capable of regenerating hematopoiesis in vivo in said donor.

3. Method for increasing the number of circulating cells capable of regenerating hematopoiesis in vivo in a donor by administration of a composition comprising the growth hormone or one of its derivatives or any factor inducing the growth hormone release to said donor.

4. Method according to any one of the preceding claims wherein the circulating cells capable of regenerating hematopoiesis in vivo are CD34⁺ cells.

5. Method according to claim 4 wherein the increased number of CD34⁺ cells is more than 10 CD34⁺ cells per microliter of peripheral blood.

6. Method according to any one of the preceding claims wherein the increased number of CD34⁺ cells is at least 2x10⁶ CD34⁺ cells per kilogram of recipient body weight, or at least 4x10⁶ CD34⁺ cells per kilogram of recipient body weight or at least 8x10⁶ CD34⁺ cells per kilogram of recipient body weight.

7. Method according to any one of the preceding claims wherein the increased number of CD34⁺ cells is at least 2x10⁶ CD34⁺ cells per kilogram of donor body weight, at least 4x10⁶ CD34⁺ cells per kilogram of donor body weight or at least 8x10⁶ CD34⁺ cells per kilogram of donor body weight.

8. Method according to any one of the preceding claims wherein the increased number of circulating cells capable of regenerating hematopoiesis in vivo corresponds to around or more than 1x10⁵ GM-CFC per kilogram of donor or recipient body weight.

9. Method according to any one of the preceding claims wherein the increased number of circulating cells capable of regenerating hematopoiesis in vivo corresponds to around or more than 500 CFU-GM per milliliter of peripheral blood.

10. Method according to to any one of the preceding claims wherein the increased number of circulating cells capable of regenerating hematopoiesis in vivo corresponds to an increased level of CFU-C, CFU-Meg or BFU-E.

11. Method according to any one of the preceding claims wherein the increased number of circulating cells capable of regenerating hematopoiesis in vivo substantially corresponds to a white blood cell count of around or more than 1000 cells per microliter of peripheral blood.

12. Method according to any one of the preceding claims wherein the circulating cells capable of regenerating hematopoiesis in vivo are CD34⁺/CD33⁺ cells and/or CD34⁺/CD38⁻ cells and/or CD34⁺/Thy-I cells and/or CD34⁺/Thy-I/CD38⁻ cells and/or CD33⁺ cells and/or bone-marrow stem cells and/or progenitor cells and/or long-term culture initiating cells (LTC-IC) and/or cells that fulfill self renewal potential and/or cells that fulfill pluripotential characteristics and/or cells that initiate long term bone marrow culture and/or cells that can generate multiple cell lineages.

13. Method of preparation of a population of circulating cells capable of regenerating hematopoiesis in vivo comprising :
a) administering to a donor a composition comprising growth hormone or one of its derivatives or any factor inducing growth hormone release in an amount sufficient to reduce the volume of blood required to be processed in order to obtain the specified target number of circulating cells capable of regenerating hematopoiesis in vivo.
b) isolating said reduced volume of blood
c) isolating a population of circulating cells capable of regenerating hematopoiesis in vivo from said isolated volume.

14. Method of preparation of a donor of circulating cells, which cells are capable of regenerating hematopoiesis in vivo comprising the administration to said donor of a composition comprising growth hormone or one of its derivatives or any factor inducing growth hormone release in an amount sufficient to reduce the volume of blood required to be processed in order to obtain the specified target number of circulating cells capable of regenerating hematopoiesis in vivo and/or to reduce the number of leukapheresis required to collect sufficient amount of circulating cells capable of regenerating hematopoiesis in vivo to be tranplanted.

15. Method for reducing the volume of blood required to be processed in order to obtain the specified target number of circulating cells capable of regenerating hematopoiesis in vivo in a donor and/or for reducing the number of leukapheresis required to collect sufficient amount of circulating cells capable of regenerating hematopoiesis in vivo to be tranplanted by administration of a composition comprising the growth hormone or one of its derivatives or any factor inducing the growth hormone release to said donor .

16. Method according to any one of the preceding claims wherein the specified target number of circulating cells capable of regenerating hematopoiesis in vivo is at least 2x10⁴ LTC-IC per kg of donor or recipient body, around or more than 2x10⁶ CD34⁺ cells per kilogram of donor or recipient body weight, around or more than 4x10⁶ CD34⁺ cells per kilogram of donor or recipient body weight or around or more than 8x10⁶ CD34⁺ cells per kilogram of donor or recipient body weight.

17. Method according to any one of the preceding claims wherein the required volume of blood is comprised in a range of about 30 to about 900 milliliters.

18. Method according to any one of the preceding claims wherein the composition comprises further one or several compound chosen among the following groups of compounds : hematopoietic growth factors, cytokines, chemokines, monoclonal antibodies.

19. Method according to any one of the preceding claims wherein the cytokine group comprises IL-1,IL-3, G-CSF, GM-CSF or SCF ; the chemokine group comprises MIP-1α or thrombopoietin (TPO) ; the monoclonal antibody group comprises anti-VLA-4 antibodies.

20. Method according to any one of the preceding claims wherein the composition comprises Growth Hormone and G-CSF.

21. Method according to claims 19 or 20 wherein the G-CSF is administered in an amount of 10µg per kilogram of body weight.

22. Method according to any one of the preceding claims wherein the administration is made by the intravenous route.

23. Method according to any one of the preceding claims wherein the administration is made by parenteral, subcutaneous, intravenous, intramuscular, intraperitoneal, transdermal or buccal routes.

24. Method according to any one of the preceding previous claims wherein the administration is daily.

25. Method according to any one of the preceding claims wherein the administration is made over a period of 5 days or over a period of 10 days.

26. Method according to any one of the preceding claims wherein the growth hormone is recombinant growth hormone.

27. Method according to any one of the preceding claims wherein the growth hormone is human growth hormone.

28. Use of human growth hormone or one of its derivatives or any factor inducing human growth hormone release to prepare a medicament for enhancing hematopoietic reconstitution in a human being.

29. Use of human growth hormone or one of its derivatives or any factor inducing human growth hormone release to prepare a medicament for enhancing hematopoietic reconstitution following bone marrow transplantation in a human being.

30. Use of human growth hormone or one of its derivatives or any factor inducing human growth hormone release to prepare a medicament for enhancing engrafment of bone marrow in a human being.

31. Use of growth hormone or one of its derivatives or any factor inducing growth hormone release to prepare a medicament for enhancing hematopoietic reconstitution after transplantation of circulating cells capable of regenerating hematopoiesis in vivo.

32. Use of growth hormone or one of its derivatives or any factor inducing growth hormone release to prepare a medicament for enhancing engrafment of circulating cells capable of regenerating hematopoiesis in vivo.

33. Use according to any one of claims 31 or 32 wherein growth hormone is human growth hormone.

34. Use according to any one of claims 28-33 wherein the hematopoietic reconstitution, or the enhanced engrafment is detected by an increase of the peripheral White Blood Cell (WBC) count and/or granulocytes count and/or lymphocytes count and/or platelet count and/or erythrocyte count.

35. Use according to any one of claims 28-34 wherein the hematopoietic reconstitution or enhanced engrafment is detected by a reduction of the period of time necessary to recover a normal peripheral White Blood Cell (WBC) count and/or granulocytes count and/or neutrophil count and/or lymphocytes count and/or platelet count and/or erythrocyte count.

36. Use according to claim 35 wherein the normal neutrophil count is at least 0.5x10⁹ per liter of peripheral blood.

37. Use according to claim 35 wherein the normal platelet counts is at least 20x10⁹ per liter of peripheral blood.

38. Use according to any one of claims 28-37 wherein the hematopoietic reconstitution enhancing engrafment results in a reduction of the extent and/or duration of neutropenia and/or thrombocytopenia and/or anemia and/or hemorrhages and/or duration of prophylaxis.

39. Use according to any one of claims 28-38 wherein the hematopoietic reconstitution or the enhancing engrafment is detected by a of the duration and/or severity of fever and infections.

40. Use according to any one of claims 28-39 wherein the hematopoietic reconstitution or the enhancing engrafment is detected by a recovery of granulocytes count which is at least 1000 per microliter of peripheral blood.

41. Use according to any one of claims 28-40 wherein the hematopoietic reconstitution or the enhancing engrafment is detected by a recovery of platelet count which is at least 50,000 per microliter of peripheral blood.

42. Use of growth hormone or one of its derivatives or any factor inducing growth hormone release to prepare a medicament for treating a neoplastic disease, an hematological disorder, malignancies, Severe Combined Immune Deficiencies (SCIDs), congenitally or genetically determined hematopoietic abnormalities, anemia, aplastic anemia, leukemia and/or osteopetrosis.

43. Use of growth hormone or one of its derivatives or any factor inducing growth hormone release to prepare a medicament for reducing the bone marrow aplasia period which follows transplantation, for preventing and/or treating opportunistic infections after transplantation or for limiting the risk of tumor recurrence after transplantation.

44. Use of growth hormone or one of its derivatives or any factor inducing growth hormone release to prepare a medicament for preventing and/or treating secondary effects of myelosuppressive therapy and/or radiotherapy and/or chemotherapy.

45. Use of growth hormone or one of its derivatives or any factor inducing growth hormone release to prepare a medicament for preventing and/or treating neutropenia and/or thrombocytopenia.

46. Use of growth hormone or one of its derivatives or any factor inducing growth hormone release to prepare a medicament for preventing or treating anemia and/or neutropenia and/or thrombocytopenia following radiotherapy and/or chemotherapy and /or hematopoietic stem cells transplantation and/or transplantation of cells capable of regenerating hematopoiesis and/or Bone marrow transplantation.

47. Use according to any one of claims 28-46 wherein the circulating cells capable of regenerating hematopoiesis in vivo are CD34⁺ cells and/or CD34⁺CD33⁺ cells and/or CD34⁺CD38⁻ cells and/or CD34⁺Thy-I cells and/or CD34⁺Thy-ICD38⁻ cells and/or CD33⁺ cells and/or stem cells and/or progenitor cells and/or long-term culture initiating cells (LTC-IC) and/or cells that fulfill self renewal potential and/or cells that fulfill pluripotential characteristics and/or cells that initiate long term bone marrow culture.

48. Use according to any one of claims 28-47 wherein the medicament comprises further one or several compound(s) chosen among the following groups of compounds : hematopoietic growth factors, cytokines, chemokines, monoclonal antibodies.

49. Use according to any one of claims 28-48 wherein the cytokines group comprises IL-1,IL-3, IL-6, IL-11, Insulin-like growth factor 1 (IGF-1), G-CSF, GM-CSF or SCF ; the chemokines group comprises MIP-1α or thrombopoietin (TPO) ; the monoclonal antibodies group comprises anti-VLA-4 antibodies.

50. Use according to any one of claims 28-49 wherein the medicament comprises Growth Hormone and G-CSF.

51. Use according to any one of claims 49 or 50 wherein the G-CSF is administrated in an amount of 10µg per kilogram.

52. Use according to any one of claims 28-51 wherein the administration is made by parenteral, subcutaneous, intravenous, intramuscular, intraperitoneal, transdermal or buccal routes.

53. Use according to any one of claims 28-52 wherein the administration is daily.

54. Use according to any one of claims 28-53 wherein the administration is made over a period of 3 days.

55. Use according to any one of claims 28-54 wherein the administration is made from day 1 to day 3 after transplantation.

56. Use according to any one of claims 28-55 wherein the transplantation is bone marrow transplantation or hematopoietic stem cells transplantation.

57. Use according to any one of claims 28-56 wherein growth hormone is recombinant growth hormone.

58. Use according to any one of claims 28-57 wherein growth hormone is human growth hormone.

59. Use according to any one of claims 28 to 58 which is preceded in time by a method according to any one of claims 1 to 27.

60. Method for the enhancement of hematopoiesis reconstitution in a human being comprising the steps of :
a) administering to a donor a composition comprising growth hormone or one of its derivatives or any factor inducing growth hormone release in an amount sufficient to increase in said donor the number of circulating cells capable of regenerating hematopoiesis in vivo.
b) isolating a population of circulating cells capable of regenerating hematopoiesis in vivo from the peripheral blood of said donor.
c) transplantation of the cells recovered in step (b) to an individual, and
d) administration of growth hormone in an amount sufficient to accelerate hematopoietic recovery.
